# EUROPEAN PATENT APPLICATION

(11) **EP 4 682 243 A1**
(43) Date of publication of application: **21.01.2026**
(21) Application number: 24821738.2
(22) Date of filing: 09.07.2024
(51) Int. Cl.: C12N 1/20, C12N 1/04, A61K 35/747, A61K 35/741, A61P 9/12

(54) **PROBIOTIC AGENT COMPRISING AKKERMANSIA MUCINIPHILA STRAIN AKK11 FOR AMELIORATING HYPERTENSION AND USE THEREOF**

(30) Priority: 30.05.2024 CN 202410686469
(71) Applicant: Wecare Probiotics Co., Ltd., Suzhou, Jiangsu 215200 (CN)
(72) Inventor: FANG, Shuguang, Suzhou, Jiangsu 215200 (CN); DONG, Yao, Suzhou, Jiangsu 215200 (CN); ZHAO, Yunjiao, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Buzzi, Notaro & Antonielli d'Oulx S.p.A.
(86) International application number: PCT/CN2024/104479
(87) International publication number: WO 2025/245966

(57) **Abstract**

Provided is a probiotic preparation containing an *Akkermansia muciniphila* Akk11 strain for alleviating hypertension, and the probiotic preparation includes an *Akkermansia muciniphila* Akk11 strain and a *Lactobacillus rhamnosus* LRa05 strain, which are found to have potential interactions between the two, and to be able to complement each other in terms of their efficacy in alleviating hypertension synergistically; specifically manifested in: significantly enhancing the ability of blood pressure regulation, and optimizing the mechanism of blood pressure regulation; significantly regulating the levels of urinary protein and serum creatinine, which are markers of renal function, effectively alleviating renal function, and alleviating renal damage caused by hypertension; and significantly optimizing the response to oxidative stress, and enhancing the antioxidant defense ability of the body.

## Description

### TECHNICAL FIELD

The present application belongs to the technical field of probiotic preparations, and relates to a probiotic preparation containing an *Akkermansia muciniphila* Akk11 strain for alleviating hypertension and use thereof.

### BACKGROUND

Traditional hypertension treatments rely primarily on medication, such as ACE inhibitors and calcium channel blockers; however, long-term drug treatment may not only trigger side effects, but may also lead to increased dependence of patients. Therefore, researchers and medical experts have been seeking safer alternatives to treatment.

Probiotics, as a biological agent that regulates the body's microecological balance, are increasingly recognized for their potential in disease prevention and treatment. Probiotic strains have been shown to have the potential to reduce blood pressure through a variety of mechanisms, such as improving gut flora balance, promoting beneficial microbial growth, reducing toxins from harmful bacteria, and enhancing gut barrier function. In addition, probiotics can also regulate the function of the autonomous nervous system by influencing the intestinal-brain axis, thus regulating blood pressure indirectly.

There are few reports and strategies on the use of second-generation probiotics such as *Akkermansia muciniphila* for anti-hypertensive purposes. Therefore, the development of more probiotic preparations that can effectively alleviate hypertension can provide a new strategy for the prevention and control of hypertension, and help to expand new use of *Akkermansia muciniphila* as well.

### SUMMARY

The present application provides a probiotic preparation containing an *Akkermansia muciniphila* Akk11 strain for alleviating hypertension and use thereof, specifically provides a probiotic preparation containing an *Akkermansia muciniphila* Akk1 1 strain for alleviating hypertension and use in the preparation of a drug for preventing, alleviating or treating hypertension thereof.

In a first aspect, the present application provides a probiotic preparation containing an *Akkermansia muciniphila* Akk11 strain for alleviating hypertension, the strain of the probiotic preparation including an *Akkermansia muciniphila* Akk11 strain and a *Lactobacillus rhamnosus* LRa05 strain; wherein the *Akkermansia muciniphila* Akk11 strain is deposited in China Center for Type Culture Collection on Jan. 15, 2024, with the deposit number of CCTCC NO. M2024119, and the address of Wuhan University, Wuhan, China; the *Lactobacillus rhamnosus* LRa05 strain is deposited in China General Microbiological Culture Collection Center on Jan. 24, 2022, with the deposit number of CGMCC No. 24377, and the address of No. 3, No. 1 West Beichen Road, Chaoyang District, Beijing, China. In the present application, a novel probiotic compounding method is developed by compounding the *Akkermansia muciniphila* Akk11 strain and the *Lactobacillus rhamnosus* LRa05 strain, which are found to have potential interactions between the two, and to be able to complement each other in terms of their efficacy in alleviating hypertension synergistically; specifically manifested in: (1) significantly enhancing the ability of blood pressure regulation, and optimizing the mechanism of blood pressure regulation; (2) significantly regulating the levels of urinary protein and serum creatinine, which are markers of renal function, effectively alleviating renal function, and alleviating renal damage caused by hypertension; (3) significantly optimizing the response to oxidative stress, and enhancing the antioxidant defense ability of the body.

In the condition of the same amount of bacteria used, compared with a single Akk11 strain or a single LRa05 strain, the compounding of the two strains has significantly alleviated the above efficacy. Therefore, the probiotic preparation provides a new strategy for preventing, relieving or treating hypertension. Because both *Akkermansia muciniphila* and *Lactobacillus rhamnosus* are probiotics, they have high safety and less dependence when used in the preparation of related efficacy products.

Preferably, a ratio of viable bacteria count of the *Akkermansia muciniphila* Akk11 strain to the *Lactobacillus rhamnosus* LRa05 strain is 1:10-10:1, for example, 1:10, 1:9, 1:8, 1:7, 1:6, 1:5, 1:4, 1. 3, 1:2, 1:1, 2:1, 3:1, 4:1, 5:1, 6:1, 7:1, 8:1, 9:1, or 10:1, etc.; other specific point values within the above numeric range are also applicable, which are not recited here for brevity.

Preferably, a total number of viable bacteria in the probiotic preparation is not less than 1 × 10⁸ CFU/mL or 1 × 10⁸ CFU/g, for example, 1 × 10⁸ CFU/mL (CFU/g), 1 × 10⁹ CFU/mL (CFU/g), 5 × 10⁹ CFU/mL (CFU/g), 1 × 10¹⁰ CFU/mL (CFU/g), 5 × 10¹⁰ CFU/mL (CFU/g), 1 × 10¹¹ CFU/mL (CFU/g), 1 × 10¹² CFU/mL (CFU/g) or 1 × 10¹³ CFU/mL (CFU/g) , etc.; other specific point values within the above numeric range are also applicable, which are not recited here for brevity.

Preferably, a dosage form of the probiotic preparation includes a solution agent, a lyophilized powder agent, a capsule agent, a tablet agent or a granule agent. The dosage form of the probiotic preparation involved in the present application is not limited and includes the most commonly used solution agent and lyophilized powder agent, or further prepared capsule agent, tablet agent or granule agent.

Preferably, the dosage form of the probiotic preparation is a solution agent, which is prepared by the following method:
inoculating the Akk11 strain and LRa05 strain in a culture medium respectively, and performing activation and fermentation culture in sequence to obtain fermentation broths; centrifuging the fermentation broths and resuspending by a solvent, respectively, to obtain an Akk11 strain suspension and a LRa05 strain suspension; and mixing the Akk11 strain suspension and the LRa05 strain suspension according to the ratio of viable bacteria count to obtain the probiotic preparation.

Preferably, the dosage form of the probiotic preparation is a lyophilized powder agent, which is prepared by the following method:
inoculating the Akk11 strain and LRa05 strain in a culture medium respectively, and performing activation and fermentation culture in sequence to obtain fermentation broths; centrifuging the fermentation broths, after mixing with a protective agent and lyophilizing, to obtain Akk11 bacteria powder and LRa05 bacteria powder; and mixing the Akk11 bacteria powder and the LRa05 bacteria powder according to the ratio of viable bacteria count to obtain the probiotic preparation.

Preferably, the protective agent is selected from any one or a combination of at least two of skimmed milk, gelatin, dextrine, Arabic gum, dextran, sodium algae, polyvinyl pyrrolidone, sucrose, lactose, trehalose, sorbitol or xylitol.

In a second aspect, the present application provides use of the probiotic preparation as described in the first aspect in the preparation of a drug for preventing, alleviating or treating hypertension.

Preferably, the drug further includes an adjuvant, and the adjuvant is selected from any one or a combination of at least two of a filler, an adhesive, a wetting agent, a disintegrator, an emulsifier, a cosolvent, a solubilizer, an osmotic pressure regulator, a colorant, a pH regulator, an antioxidant, an antimicrobial agent or a buffer.

Preferably, the drug further includes a functional additive, and the functional additive is selected from any one or a combination of at least two of oligofructose, oligogalactose, oligoxylose, inulin, Coriolus versicolor polysaccharide, polyglucose, α-lactalbumin, or lactoferrin.

Compared with the prior art, the present application has the following beneficial effects.

In the present application, a novel probiotic compounding method is developed by compounding the *Akkermansia muciniphila* Akk11 strain and the *Lactobacillus rhamnosus* LRa05 strain, which are found to have potential interactions between the two, and to be able to complement each other in terms of their efficacy in alleviating hypertension synergistically; specifically manifested in: (1) significantly enhancing the ability of blood pressure regulation, and optimizing the mechanism of blood pressure regulation; (2) significantly regulating the levels of urinary protein and serum creatinine, which are markers of renal function, effectively alleviating renal function, and alleviating renal damage caused by hypertension; (3) significantly optimizing the response to oxidative stress, and enhancing the antioxidant defense ability of the body.

In the condition of the same amount of bacteria used, compared with a single Akk11 strain or a single LRa05 strain, the compounding of the two strains has significantly alleviated the above efficacy. Therefore, the probiotic preparation provides a new strategy for preventing, relieving or treating hypertension. Because both *Akkermansia muciniphila* and *Lactobacillus rhamnosus* are probiotics, they have high safety and less dependence when used in the preparation of related efficacy products.

### BRIEF DESCRIPTION OF DRAWINGS

FIG. 1 is a statistical result chart for caudal artery systolic pressures of rats in each group.
FIG. 2 is a statistical result chart for norepinephrine contents in plasma of rats in each group.
FIG. 3 is a statistical result chart for adrenaline contents in plasma of rats in each group.
FIG. 4 is a statistical result chart for creatinine contents in serum of rats in each group.
FIG. 5 is a statistical result chart for urinary protein contents in urine of rats in each group.
FIG. 6 is a statistical result chart for malondialdehyde levels in renal tissue of rats in each group.
FIG. 7 is a statistical result chart for superoxide dismutase levels in renal tissue of rats in each group.
FIG. 8 is a statistical result chart for glutathione levels in renal tissue of rats in each group.

### DETAILED DESCRIPTION

The technical solutions of the present application are further described below in terms of specific embodiments. It should be clear to those skilled in the art that the embodiments are merely used for a better understanding of the present application and should not be regarded as a specific limitation to the present application.

Formulation to prepare the medium involved in the following examples is as follows:
MRS medium: 10 g/L of peptone, 10 g/L of beef paste, 20 g/L of glucose, 2 g/L of sodium acetate, 5 g/L of yeast powder, 2 g/L of diammonium hydrogen citrate, 2.6 g/L of K₂PO₄·3H₂O, 0.1 g/L of MgSO₄·7H₂O, 0.05 g/L of MnSO₄, 80 1 mL/L of polysorbate, 0.5 g/L of cysteine hydrochloride.

The binomial nomenclature of the Akk11 strain involved in the following examples is *Akkermansia muciniphila* Akk11, which is deposited in China Center for Type Culture Collection on Jan. 15, 2024, with the deposit number of CCTCC NO. M2024119, and the address of Wuhan University, Wuhan, China.

The binomial nomenclature of the LRa05 strain involved in the following examples is *Lactobacillus rhamnosus,* which is deposited in China General Microbiological Culture Collection Center on Jan. 24, 2022, with the deposit number of CGMCC No. 24377, and the address of No. 3, No. 1 West Beichen Road, Chaoyang District, Beijing, China.

The preparation method of a bacterial suspension involved in the following: the required strain was inoculated in a liquid culture medium, cultured at 37°C for 24 h for activation, the activation was performed twice continuously to obtain an activation solution; the activation solution was inoculated in a liquid culture medium at an inoculate dosage of 5% (v/v), cultured at 37°C for 24 h to obtain a bacterial solution; the bacterial solution was centrifuged at 4°C for 10 min with a rotational speed of 5000 rpm, filtered to obtain a bacterial pellet; the bacterial pellet were resuspended by a PBS solution to obtain the bacterial suspension.

The data of the test results were statistically analyzed using ggplot2 in R Programming Language. Compared with the control group, #### stands for p < 0.001; compared with the model group, *** stands for p < 0.001, ** stands for p < 0.01, * stands for p < 0.05, and NS. stands for no significant difference.

### Example

This example explores the ability of strains to alleviate symptoms of hypertensive rat model.
(1) Test animals: healthy Dahl salt-sensitive rats of SPF grade, male, 8 weeks old (56 rats, purchased from Shanghai Chengxi Biotech. Co., Ltd); the rats were reared in a controlled environment with room temperature of 20-24°C and humidity of 50-60%, following a 12 h light/dark cycle, freely to eat and drink.
(2) Animal grouping: after 1 week of adaptive feeding, rats were randomly assigned to 7 groups, 8 in each group: control group (CTL), high-salt model group (MC group), Akk11 strain group (Akk11 group, recorded as S1 group), LRa05 strain Group (LRa05 group, recorded as S2 group), marketed *Akkermansia muciniphila* group (BNCC341917 group, recorded as S3 group), complex strain group 1 (Akk11 + LRa05 group, the ratio of viable bacteria count was 1:1, recorded as S4 group), complex strain group 2 (BNCC341917 + LRa05 group, the ratio of viable bacteria count was 1:1, recorded as S5 group).
(3) Animal modeling and intervention method
   Rats in the CTL group were fed with 0.3% sodium chloride-containing feed, and rats in the MC group and probiotic intervention group were fed with 8% sodium chloride-containing feed to establish a salt-sensitive hypertension model. In addition, the rats in the intervention group also had an additional oral probiotic bacteria solution (total bacterial dose 10⁸ CFU/day/individual), and all groups of rats intervened for 4 weeks. During the experiment, the caudal artery systolic pressure of rat increased and renal fibrosis appeared, indicating that the model was established successfully.
(4) Indicator analysis
   (4.1) Effect on caudal artery systolic pressure of rats

After intervention, the caudal artery systolic pressure of rats in calm state of each group was measured using a multi-parameter monitor at the same time period of 8-9 am.

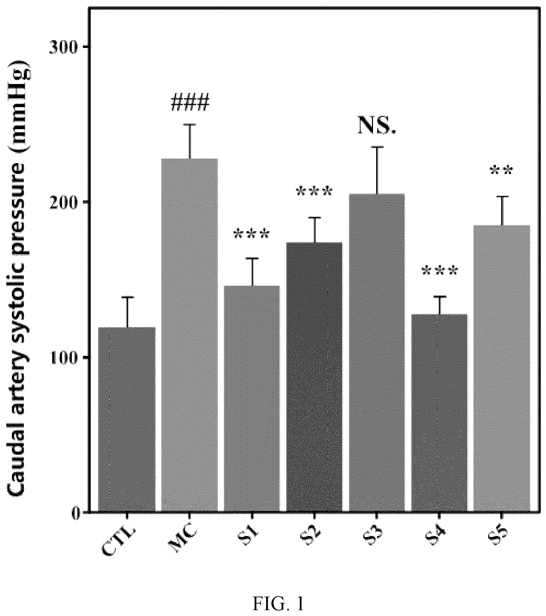

The results are shown in FIG.1. As can be seen from the figure" compared with the CTL group, the caudal artery systolic pressure of rats in the MC group is significantly increased, indicating that the model successfully induce a hypertensive state. After receiving probiotic intervention in each group, the caudal artery systolic pressure of rats is significantly decreased, especially in S4 group where the effect is most significant, which suggests that the intervention of the probiotic preparation of the present application can alleviate the elevated blood pressure induced by the hypertension model, and the Akk11 strain and the LRa05 strain have potential synergistic effects in the above efficacy.

### (4.2) Effect on the contents of norepinephrine and epinephrine in plasma of rats

After intervention, 200 µL of blood was collected from each rat at a collection time of 10-11 am, and plasma was collected from each group of rats. Contents of norepinephrine (NE) and epinephrine (EPI) in plasma were measured and calculated according to the instructions of ELISA kit.

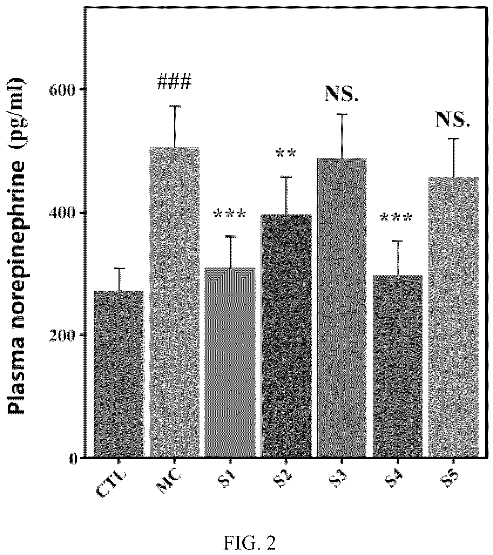

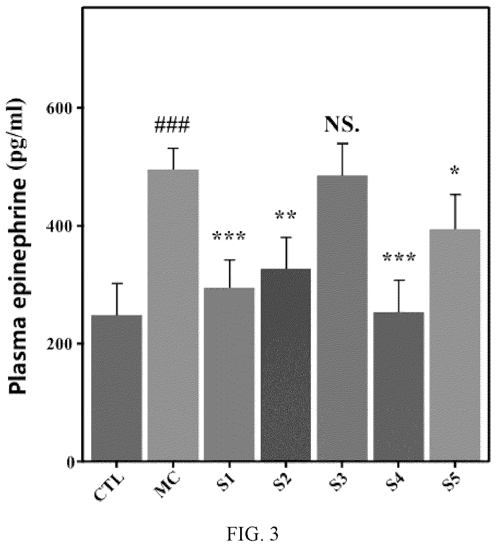

The results are shown in FIG. 2 and FIG. 3. As can be seen from the figures,, compared with the CTL group, the contents of norepinephrine (NE) and epinephrine (EPI) in plasma is significantly increased of rats in the MC group, which is in line with the typical physiological reaction of increased sympathetic nerve activities under hypertension. After the probiotic intervention, the levels of these two important stress hormones is significantly decreased, which is close to the level of healthy control group,, indicating that the probiotic involved in the present application has the potential to modulate endocrine reaction and thus alleviate hypertension symptoms. Especially in S4 group, this reduction effect is the most significant.

### (4.3) Effect on the contents of serum creatinine of rats

After intervention, rats were anesthetized by intraperitoneal injection of 10% chloral hydrate, and 5 mL of blood was taken from the abdominal aorta, centrifuged at 4000 r/min for 10 min, serum was separated, and the level of serum creatinine was measured by a serum creatinine assay kit.

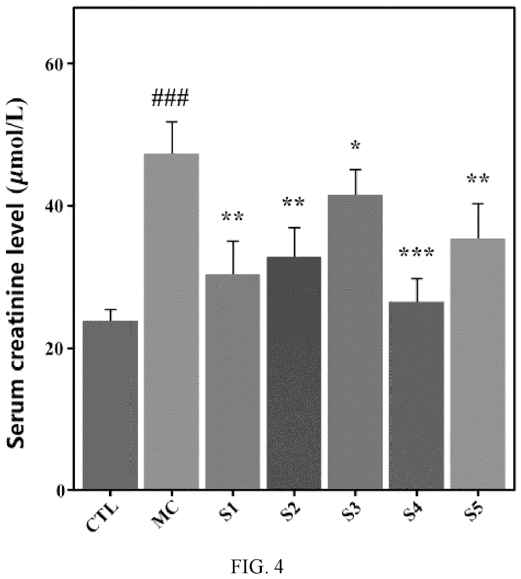

The results are shown in FIG. 4. As can be seen from the figure,, the rats in MC group have obvious renal function damage under hypertension, specifically manifested as a significant increase in serum creatinine level, which is significantly different from that in CTL group. After the probiotic intervention, the serum creatinine level of rats is significantly decreased, indicating that the probiotic has the potential ability to protect renal function and improve renal health. Especially in S4 group, this reduction effect was more prominent, more effectively alleviating the renal damage caused by hypertension.

### (4.4) Effect on the contents of urinary protein of rat

After intervention, urine was collected from each group of rats for 24 h, and urinary protein was detected using a urine protein quantitative assay kit.

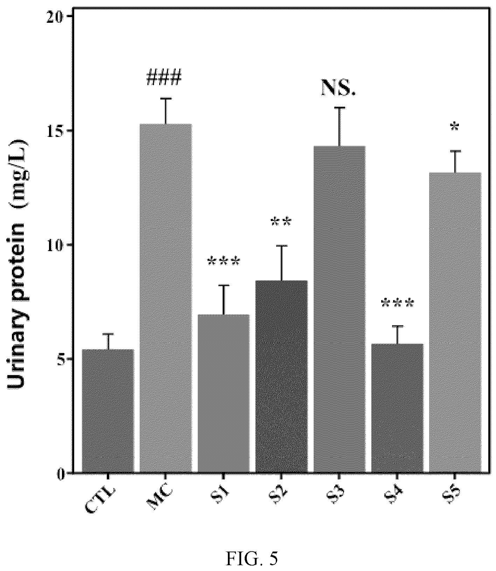

The results are shown in FIG. 5. As can be seen from the figure" the rats in MC group have obvious renal function damage under hypertension, specifically manifested as a significant increase in urinary protein level, which is significantly different from that in CTL group. After the probiotic intervention, the urinary protein level of the rats is significantly decreased, indicating that the probiotic has the potential ability to protect renal function and improve renal health. Especially in S4 group, this reduction effect was more prominent, more effectively alleviating the renal damage caused by hypertension.

### (4.5) Effect on oxidative stress indexes

After intervention, rats were killed and the right renal tissue was taken, and the supernatant of the tissue homogenate was prepared. According to the instructions of a malondialdehyde, superoxide dismutase, and glutathione assay kit, the levels of malondialdehyde, superoxide dismutase, and glutathione in rat renal tissues were detected.

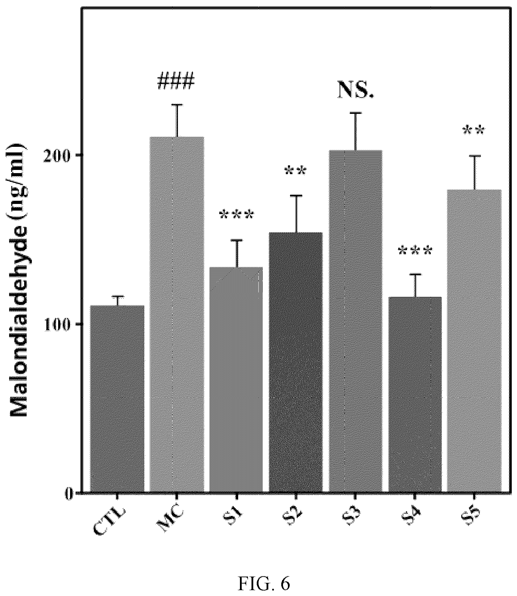

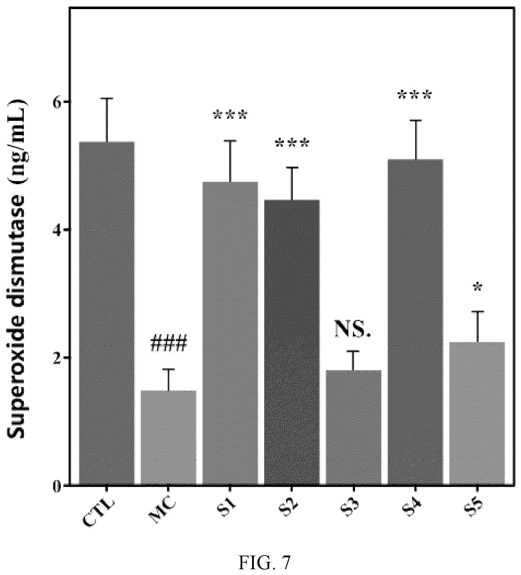

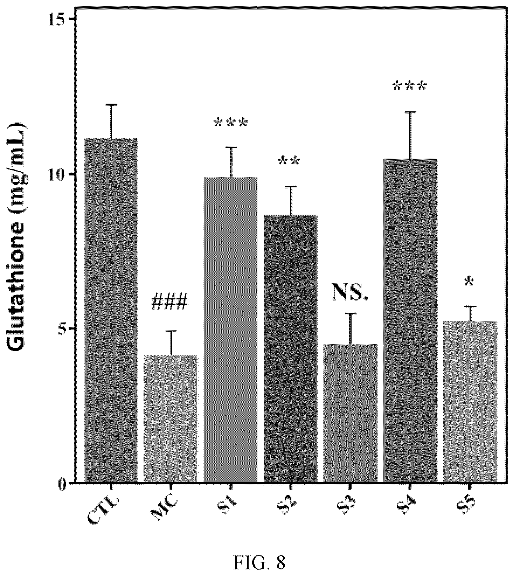

The results are shown in FIG. 6, FIG. 7 and FIG. 8. Under the condition of oxidative stress induced by hypertension, the level of malondialdehyde (MDA) is significantly increased, while the levels of superoxide dismutase (SOD) and glutathione (GSH) are significantly decreased in MC group, which reflects the intensification of oxidative stress and the weakening of the antioxidant defense mechanism. After the probiotic intervention, the level of malondialdehyde (MDA) is significantly decreased, while the levels of superoxide dismutase (SOD) and glutathione (GSH) are significantly increased in hypertensive rats, indicating that the probiotic effectively alleviated the oxidative damage induced by hypertension. Especially in S4 group, these effects are more significant, which shows that the probiotic preparation involved in the present application has played an important role in promoting the antioxidant defense system and are helpful to restore the antioxidant balance of cells.

The applicant declares that the present application illustrates the detailed technical solutions of the present application by the above examples, but the present application is not limited to the above examples, that is, the present application does not necessarily rely on the above examplesto be implemented. Those skilled in the art should understand that any improvements of the present application, the equivalent substitution of each raw material, the addition of auxiliary ingredients, and the selection of specific methods shall fall within the protection scope and disclosure scope of the present application.

The above describes in detail the preferred embodiments of the present application. However, the present application is not limited to the specific details in the above embodiments, and various simple variations of the technical solutions of the present application can be made within the scope of the technical conception of the present application, all of these simple variations shall fall within the protection scope of the present application.

It is also to be noted that the various specific technical features described in the above specific embodiments may be combined in any suitable manners without contradiction, and in order to avoid unnecessary repetition, the various possible combinations are not described separately in the present application.

## Claims

1. A probiotic preparation containing an *Akkermansia muciniphila* Akk11 strain for alleviating hypertension, comprising an *Akkermansia muciniphila* Akk11 strain with the deposit number of CCTCC NO: M2024119, and a *Lactobacillus rhamnosus* LRa05 strain with the deposit number of CGMCC No.24377.

2. The probiotic preparation according to claim 1, wherein a ratio of viable bacteria count of the *Akkermansia muciniphila* Akk11 strain to the *Lactobacillus rhamnosus* LRa05 strain is 1:10-10:1.

3. The probiotic preparation according to claim 1, wherein a total number of viable bacteria in the probiotic preparation is more than or equal to 1 × 10⁸ CFU/mL or 1 × 10⁸ CFU/g.

4. The probiotic preparation according to claim 1, wherein a dosage form of the probiotic preparation comprises a solution agent, a lyophilized powder agent, a capsule agent, a tablet agent or a granule agent.

5. The probiotic preparation according to claim 4, wherein the dosage form of the probiotic preparation is a solution agent, which is prepared by the following method:
inoculating the Akk11 strain and LRa05 strain in a culture medium respectively, and performing activation and fermentation culture in sequence to obtain fermentation broths; centrifuging the fermentation broths and resuspending by a solvent, respectively, to obtain an Akk11 strain suspension and a LRa05 strain suspension; and mixing the Akk11 strain suspension and the LRa05 strain suspension according to the ratio of viable bacteria count to obtain the probiotic preparation.

6. The probiotic preparation according to claim 4, wherein the dosage form of the probiotic preparation is a lyophilized powder agent, which is prepared by the following method:
inoculating the Akk11 strain and LRa05 strain in a culture medium respectively, and performing activation and fermentation culture in sequence to obtain fermentation broths; centrifuging the fermentation broths, after mixing with a protective agent and lyophilizing, to obtain Akk11 bacteria powder and LRa05 bacteria powder; and mixing the Akk11 bacteria powder and the LRa05 bacteria powder according to the ratio of viable bacteria count to obtain the probiotic preparation.

7. The probiotic preparation according to claim 6, wherein the protective agent is selected from any one or a combination of at least two of skimmed milk, gelatin, dextrine, Arabic gum, dextran, sodium algae, polyvinyl pyrrolidone, sucrose, lactose, trehalose, sorbitol or xylitol.

8. Use of the probiotic preparation according to any one of claims 1-7 in the preparation of a drug for preventing, alleviating or treating hypertension.

9. The use according to claim 8, wherein the drug further comprises an adjuvant, and the adjuvant is selected from any one or a combination of at least two of a filler, an adhesive, a wetting agent, a disintegrator, an emulsifier, a cosolvent, a solubilizer, an osmotic pressure regulator, a colorant, a pH regulator, an antioxidant, an antimicrobial agent or a buffer.

10. The use according to claim 8, wherein the drug further comprises a functional additive, and the functional additive is selected from any one or a combination of at least two of oligofructose, oligogalactose, oligoxylose, inulin, Coriolus versicolor polysaccharide, polyglucose, α-lactalbumin, or lactoferrin.
